(19) Europäisches Patentamt
European Patent Office
Office européen des brevets

(11) **EP 2 256 110 A1**

(12) **EUROPÄISCHE PATENTANMELDUNG**

(43) Veröffentlichungstag:
**01.12.2010 Patentblatt 2010/48**

(51) Int Cl.:
*C07D 301/12* (2006.01)      *C01B 37/00* (2006.01)

(21) Anmeldenummer: **10173947.2**

(22) Anmeldetag: **10.05.2004**

(84) Benannte Vertragsstaaten:
**AT BE BG CH CY CZ DE DK EE ES FI FR GB GR
HU IE IT LI LU MC NL PL PT RO SE SI SK TR**

(30) Priorität: **08.05.2003 DE 10320635**

(62) Dokumentnummer(n) der früheren Anmeldung(en)
nach Art. 76 EPÜ:
**04731925.6 / 1 625 118**

(71) Anmelder: **BASF SE
67056 Ludwigshafen (DE)**

(72) Erfinder:
  • **Müller, Ulrich
    67435, Neustadt (DE)**
  • **Krug, Georg
    69509, Mörlenbach (DE)**

• **Rudolf, Peter
  68526, Ladenburg (DE)**
• **Teles, Joaquim Henrique
  67166, Otterstadt (DE)**
• **Göbbel, Hans-Georg
  67169, Kallstadt (DE)**
• **Baßler, Peter
  68519, Viernheim (DE)**

(74) Vertreter: **Altmann, Andreas
  Herzog Fiesser & Partner
  Patentanwälte
  Isartorplatz 1
  80331 München (DE)**

Bemerkungen:
  Diese Anmeldung ist am 25-08-2010 als
  Teilanmeldung zu der unter INID-Code 62 erwähnten
  Anmeldung eingereicht worden.

(54) **Verfahren zur Herstellung von Propylenoxid**

(57)  Verfahren zur Herstellung von Propylenoxid, mindestens umfassend die Schritte (i) und (ii):
(i) Bereitstellen eines Katalysators, enthaltend mindestens ein poröses oxidisches Material;
(ii) Umsetzung von Propen mit einem Hydroperoxid in mindestens einem Nitril als Lösungsmittel oder in einem mindestens ein Nitril enthaltenden Lösungsmittelgemisch in Anwesenheit des Katalysators gemäß (i),

**dadurch gekennzeichnet, dass** das mindestens ein poröse oxidische Material ein Zcolith mit röntgenographischer Zuordnung zum MWW-Typ ist.

**EP 2 256 110 A1**

**Beschreibung**

Die vorliegende Erfindung betrifft ein Verfahren zur Herstellung von Propylenoxid, bei dem Propen mit einem Hydroperoxid in einem Lösungsmittel, das entweder ein Nitril ist oder ein Nitril enthält, umgesetzt wird. Katalysiert wird diese Umsetzung von einem Zeolith-Katalysator, wobei der Zeolith vom röntgenographischen Strukturtyp MWW ist. Ganz allgemein betrifft die vorliegende Erfindung die Verwendung eines Zeolithkatalysators vom röntgenographischen Strukturtyp MWW zur Epoxidierung von Propen mit einem Hydroperoxid.

Die Epoxidierung von Olefinen unter Verwendung von Hydroperoxiden in Anwesenheit eines Katalysators und mindestens eines Lösungsmittels ist in vielen Schriften des Standes der Technik behandelt.

So beschreibt beispielsweise die WO 02/28774 A2 ein Verfahren, bei dem zur Epoxidierung einer Verbindung, die eine C-C-Doppelbindung aufweist, ein Zeolithkatalysator vom Strukturtyp MWW eingesetzt wird. Dabei müssen die umzusetzenden Verbindungen neben der C-C-Doppelbindung zwingend mindestens eine weitere funktionelle Gruppe enthalten. Als solche weiteren funktionellen Gruppen sind neben einer Reihen weiterer Gruppen unter anderem Halogen-, Aldehyd-, Keton-, Ester-, Amid- oder auch Amingruppen offenbart. Für diese katalytischen Reaktionen wird im Rahmen der WO 02/28774 A2 lediglich das poröse Material des Zeolithen eingesetzt, Katalysatorformkörper werden hingegen aus dem porösen Material nicht hergestellt.

Wu et al. beschreiben in "Hydrothermal Synthesis of a novel Titanosilicate with MWW Topology", Chemistry Letters 2000, S. 774-775, die Herstellung eines neuartigen Titansilikaliten mit MWW-Struktur. Dabei wird dieser Silikalit direkt unter Verwendung von Bor als strukturbildendem Mittel hergestellt. Der so hergestellte und Titan enthaltende MWW-Zeolith wird hinsichtlich seiner katalytischen Aktivität bei der Flüssigphasenoxidation von Alkenen getestet. Für diese katalytischen Tests wird lediglich das poröse Material des Zeolithen eingesetzt, Katalysatorformkörper werden hingegen aus dem porösen Material nicht hergestellt. Als einzig explizit offenbartes Alken, das mittels des MWW-Zeolithen epoxidiert wird, wird in Wu et al. Cyclohexen beschrieben. Wu et al. beschreiben Cyclohexen hierbei in einer Reihe mit Toluol, das mit MWW-Zeolith-Katalyse disproportioniert wurde, oder Benzol, das mit MWW-Zeolith-Katalyse alkyliert wurde. Mit diesem MWW-Aktivmaterial werden bezüglich der Umsetzung von Cyclohexen mit Wasserstoffperoxid Selektivitäten hinsichtlich der unerwünschten Folgeprodukte der Glykole von mindestens 28 % in Kauf genommen, bei der Umsetzung mit tert-Butylhydroperoxid von immerhin mindestens 4 %.

Eine der der vorliegenden Erfindung zugrunde liegenden Aufgaben war es daher, ein Verfahren bereitzustellen, mit dem ein Alken so umgesetzt werden kann, dass die Selektivität hinsichtlich der unerwünschten Glykolfolgeprodukte geringer ist als im Stand der Technik beschrieben.

Demgemäß betrifft die vorliegende Erfindung ein Verfahren zur Herstellung von Propylenoxid, mindestens umfassend die Schritte (i) und (ii):

(i) Bereitstellen eines Katalysators, enthaltend mindestens ein poröses oxidisches Material;

(ii) Umsetzung von Propen mit einem Hydroperoxid in mindestens einem Nitril als Lösungsmittel oder in einem mindestens ein Nitril enthaltenden Lösungsmittelgemisch in Anwesenheit des Katalysators gemäß (i),

**dadurch gekennzeichnet, dass** das mindestens ein poröse oxidische Material ein Zeolith mit röntgenographischer Zuordnung zum MWW-Typ ist.

Der Umsatz an Hydroperoxid, mit dem Propen umgesetzt wird, liegt im erfindungsgemäßen Verfahren bei mindestens 90 %.

Bezogen auf Hydroperoxid liegt die Selektivität im erfindungsgemäßen Verfahren hinsichtlich des Verfahrensproduktes Propylenoxid bei mindestens 80 %, bevorzugt bei mindestens 85 % und besonders bevorzugt bei mindestens 88 %.

Bezogen auf das Edukt Hydroperoxid liegt die Selektivität im erfindungsgemäßen Verfahren hinsichtlich der entstehenden Epoxidfolgeprodukte bei höchstens 10 %, weiter bevorzugt bei höchstens 8 %, weiter bevorzugt bei höchstens 6 % und besonders bevorzugt bei höchstens 5 %.

Demgemäß betrifft die vorliegende Erfindung auch ein Verfahren, wie oben beschrieben, das dadurch gekennzeichnet ist, dass die Selektivität hinsichtlich der Epoxidfolgeprodukte, bezogen auf Hydroperoxid, höchstens 10 % beträgt.

Der Begriff "Epoxidfolgeprodukt", wie er im Rahmen der vorliegenden Erfindung verwendet wird, bezeichnet sämtliche denkbaren Folgeprodukte, die bei der Umsetzung gemäß (ii) in dem Reaktor, in dem die Umsetzung des Propens mit dem Edukt Hydroperoxid erfolgt, aus dem gebildeten Propylenoxid entstehen können. Insbesondere sind dies Glykole wie beispielsweise Propylenglykol und Polyetherpolyole und/oder Hydroperoxide, die aus dem gebildeten Propylenoxid und dem eingesetzten Hydroperoxid wie beispielsweise Wasserstoffperoxid entstehen.

Hinsichtlich der Glykolfolgeprodukte aus der Gruppe der Epoxidfolgeprodukte liegt die Selektivität bei höchstens 5 %, bevorzugt bei höchstens 4 % weiter bevorzugt bei höchstens 3 % und besonders bevorzugt bei höchstens 2 %.

Der Katalysator, der gemäß (i) bereitgestellt und gemäß (ii) zur Umsetzung eingesetzt wird, enthält ein poröses oxidisches Material, das wiederum ein Zeolith eines bestimmten Strukturtyps ist. Zeolithe sind bekanntermaßen kristalline Alumosilikate mit geordneten Kanal- und Käfigstrukturen, die beispielsweise Mikroporen aufweisen, die vorzugsweise kleiner als ungefähr 0,9 nm sind. Das Netzwerk solcher Zeolithe ist aufgebaut aus $SiO_4$- und $AlO_4$-Tetraedern, die über gemeinsame Sauerstoffbrücken verbunden sind. Eine Übersicht der bekannten Strukturen findet sich beispielsweise bei W.M. Meier, D.H. Olson und Ch. Baerlocher "Atlas of Zeolite Structure Types", Elsevier, 5. Auflage, Amsterdam 2001.

Ebenso sind auch Zeolithe bekannt, die kein Aluminium enthalten und bei denen im Silikatgitter anstelle des Si(IV) teilweise Titan als Ti(IV) enthalten ist. Diese Titan-Zeolithe, insbesondere solche mit einer Kristallstruktur vom MWW-Typ, sowie Möglichkeiten zu ihrer Herstellung sind beispielsweise in der oben zitierten WO 02/28774 A2 oder in dem oben zitierten Artikel von Wu et al. beschrieben, deren diesbezüglicher Inhalt durch Bezugnahme in den Kontext der vorliegenden Anmeldung aufgenommen wird. Beispielsweise sind konkrete Synthesen von Ti-MWW in den Beispielen 1 bis 5 der WO 02/28774 A2 beschrieben.

Außer Silicium und Titan können solche Materialien auch zusätzliche Elemente wie z.B. Aluminium, Zirconium, Zinn, Eisen, Kobalt, Nickel, Gallium, Germanium, Bor oder geringe Mengen an Fluor enthalten. In dem im erfindungsgemäßen Verfahren vorzugsweise verwendeten Titanzeolith-Katalysatoren kann das Titan des Zeolithen teilweise oder vollständig durch Vanadium, Zirconium, Chrom oder Niob oder einem Gemisch aus zwei mehr davon ersetzt sein. Das molare Verhältnis von Titan und/oder Vanadium, Zirconium, Chrom oder Niob zur Summe aus Silicium und Titan und/ oder Vanadium und/oder Zirconium und/oder Chrom und/oder Niob liegt in der Regel im Bereich von 0,001 : 1 bis 0,1 : 1.

Titan-Zeolithe mit MWW-Struktur sind dafür bekannt, dass sie über ein bestimmtes Muster bei der Bestimmung ihrer Röntgenbeugungsaufnahmen sowie zusätzlich über eine im Infrarot-Bereich (IR) bei $960 \pm 5$ cm$^{-1}$ befindliche Gerüstschwingungsbande identifiziert werden können und sich damit von Alkalimetalltitanaten oder kristallinen und amorphen $TiO_2$-Phasen wie etwa Rutil, Anatas oder Brookit unterscheiden.

Im Allgemeinen können im erfindungsgemäßen Verfahren sämtliche der oben beschriebenen Zeolithe vom MWW-Strukturtyp eingesetzt werden. Bevorzugt werden Titanzeolithe eingesetzt.

Demgemäß betrifft die vorliegende Erfindung auch ein Verfahren, wie oben beschrieben, das dadurch gekennzeichnet ist, dass der Zeolith vom MWW-Typ ein Titanzeolith ist.

In besonders bevorzugten Ausführungsformen weist der im erfindungsgemäßen Verfahren verwendete Titanzeolith vom MWW-Typ folgende chemische Zusammensetzungen (I) oder (II) auf:

$$x \cdot TiO_2 \, (1\text{-}x) \cdot SiO_2$$

wobei $0,0001 \leq x \leq 0,2$, oder

$$x \cdot TiO_2 \; y \cdot M_2O_3 \; (1\text{-}x\text{-}2y) \cdot SiO_2 \qquad\qquad (II)$$

wobei $0,0001 \leq x \leq 0,2$ und $0,0001 \leq y \leq 0,1$ und M mindestens ein Element aus der Gruppe bestehend aus Aluminium, Bor, Chrom, Gallium, Germanium und Eisen ist. Die Variablen x und y bezeichnen hierbei den jeweiligen molaren Anteil.

Weitere Details hinsichtlich des Strukturtyps MWW sind der oben genannten Literaturstelle W.M. Meier, D.H. Olson und Ch. Baerlocher "Atlas of Zeolite Structure Types", Elsevier, 5. Aufl., S. 202 und 203, Amsterdam 2001 zu entnehmen, deren diesbezüglicher Inhalt durch Bezugnahme in den Kontext der vorliegenden Anmeldung aufgenommen wird.

Im erfindungsgemäßen Verfahren können auch Titanzeolithkatalysatoren eingesetzt werden, die entweder mindestens zwei der Titanzeolithe gemäß (I) oder mindestens zwei der Titanzeolithe gemäß (II) oder mindestens einen Titanzeolithen gemäß (I) und mindestens einen Titanzeolithen gemäß (II) enthalten.

Grundsätzlich kann im erfindungsgemäßen Verfahren das poröse oxidische Material als solches als Katalysator eingesetzt werden. Bezüglich der Herstellung des porösen oxidischen Materials gibt es im Wesentlichen keine Einschränkungen, solange dadurch ein Zeolith und bevorzugt ein Titanzeolith vom Strukturtyp MWW erhalten wird. Zur Synthese des porösen oxidischen Materials sei beispielsweise auf die oben zitierten WO 02/28774 A2 und Wu et al. verwiesen, deren diesbezüglicher Inhalt durch Bezugnahme in die vorliegende Anmeldung vollumfänglich einbezogen wird.

Gemäß einer besonders bevorzugten Ausführungsform des erfindungsgemäßen Verfahrens wird der wie beispielsweise in WO 02/28774 A2 hergestellte Titanzeolith vom Typ MWW durch ein geeignetes Verfahren aus seiner Mutterlauge abgetrennt und weiter bevorzugt gemäß eines oder mehrerer geeigneter Verfahren getrocknet und wiederum bevorzugt anschließend calciniert. Die Calcinierung kann beispielsweise bevorzugt in einer geeigneten Gasatmosphäre erfolgen, wobei besonders bevorzugt Luft und/oder Magerluft als Gasatmosphäre verwendet wird.

Sämtliche Verfahren zur Abtrennung des Titanzeolithen aus seiner Mutterlauge sind denkbar. Unter anderem seien etwa Filtrations-, Ultrafiltrations-, Diafiltrations-, Zentrifugierverfahren oder etwa Sprühtrocknungs- und Sprühgranulierverfahren genannt. Bevorzugt wird der Titanzeolith durch Sprühtrocknung oder durch Ultrafiltration aus der Mutterlauge abgetrennt. Vor der Abtrennung des Zeolithen aus der Mutterlauge ist es möglich, den Gehalt der Mutterlauge an Zeolithen durch Aufkonzentrieren zu erhöhen. Ebenso ist es möglich, statt der Abtrennung des Zeolithen aus der Mutterlauge ausschließlich ein oder mehrere Verfahren zur Aufkonzentrierung anzuwenden. Details zur Abtrennung des Zeolithen aus der Mutterlauge sind auch in der DE 102 32 406.9 zu finden, die diesbezüglich durch Bezugnahme in den Kontext der vorliegenden Anmeldung vollumfänglich aufgenommen wird.

Im Falle, dass beispielsweise nach der Sprühtrocknung eine weitere Trocknung erwünscht ist, wird der von der Mutterlauge abgetrennte Titanzeolith bei Temperaturen im Bereich von im allgemeinen 80 bis 160 °C, bevorzugt von 90 bis 145 °C und besonders bevorzugt von 100 bis 130 °C getrocknet. Die anschließend bevorzugt erfolgende Calcinierung wird bei Temperaturen im Bereich von im allgemeinen 400 bis 750 °C, bevorzugt von 450 bis 600 °C und besonders bevorzugt von 490 bis 530 °C durchgeführt.

Gemäß weiterer Ausführungsformen des erfindungsgemäßen Verfahrens kann der Zeolith nach der Abtrennung aus der Mutterlauge mit einer Wasser enthaltenden Zusammensetzung in Kontakt gebracht werden. Dieses Inkontaktbringen kann ebenso nach der oben beschrieben Trocknung und/oder dem oben beschriebenen Calcinieren erstmals erfolgen oder wiederholt werden. In diesen Fällen können sich nach dem Inkontaktbringen mit der Wasser enthaltenden Zusammensetzung eine oder mehrere der oben beschriebenen Behandlungen zur Aufkonzentrierung oder zum Abtrennen anschließen. Als Wasser enthaltende Zusammensetzung wird beispielsweise bevorzugt Wasser an sich verwendet. Ebenso sind wässrige Aminlösungen möglich, wobei das in dieser Lösung enthaltene mindestens eine Amin Ammoniak, ein organisches aliphatisches Amin oder ein quartäres Ammoniumhydroxid sein kann und wobei der Stickstoff in diesen Stickstoffverbindungen als Alkylreste beispielsweise Methyl-, Ethyl- oder Propylreste aufweisen kann und auch zwei oder mehr unterschiedliche Alkylreste an einem Stickstoff gebunden sein können. Das Inkontaktbringen mit beispielsweise bevorzugt Wasser an sich findet im allgemeinen bei Temperaturen im Bereich von Raumtemperatur bis 750 °C, bevorzugt von 100 bis 250 °C und besonders bevorzugt von 120 bis 175 °C statt, wobei das Inkontaktbringen bevorzugt zwischen 12 und 48 h andauert. Ganz besonders bevorzugt findet dieses Inkontaktbringen in einem Autoklaven statt.

Im Falle, dass der Zeolith nach dem Abtrennen aus der Mutterlauge getrocknet und/oder calciniert wurde und anschließend mit einer Wasser enthaltenden Zusammensetzung in Kontakt gebracht wurde, kann sich ein nochmaliges Trocknen und/oder Calcinieren anschließen. Diese Trocknung erfolgt dabei bei Temperaturen im Bereich von im allgemeinen 80 bis 160 °C, bevorzugt von 90 bis 145 °C und besonders bevorzugt von 100 bis 130 °C. Die anschließend bevorzugt erfolgende Calcinierung wird bei Temperaturen im Bereich von im allgemeinen 400 bis 750 °C, bevorzugt von 450 bis 600 °C und besonders bevorzugt von 490 bis 530 °C durchgeführt.

Zusätzlich oder statt des Inkontaktbringens mit der Wasser enthaltenden Zusammensetzung kann der Zeolith noch mit beispielsweise Wasserstoffperoxidlösung, unter anderem bevorzugt schwefelsaurer Wasserstoffperoxidlösung, gewaschen werden. Ebenso ist es möglich, das zeolithische Material mit Alkalimetallionen zu behandeln, um den Zeolithen von der H-Form in die kationische Form zu bringen.

Wird der Zeolith gemäß der bevorzugten Ausführungsform nach der Abtrennung aus der Mutterlauge mit der Wasser enthaltenden Zusammensetzung in Kontakt gebracht, so weist das erhaltene zeolithische Material bei Einsatz im erfindungsgemäßen Verfahren gegenüber nicht behandelten Zeolithen den Vorteil auf, dass die Selektivität der Umsetzung hinsichtlich des unerwünschten Nebenproduktes Sauerstoff, bezogen auf Hydroperoxid, signifikant reduziert wird. Während die Sauerstoffselektivität bei nicht behandeltem Zeolith in der Regel im Bereich von 11 bis 15 % liegt, kann durch das erfindungsgemäß bevorzugte Inkontaktbringen mit der Wasser enthaltenden Zusammensetzung die Sauerstoffselektivität auf höchstens 10 %, bevorzugt auf höchstens 9, weiter bevorzugt auf höchstens 8 und besonders bevorzugt auf höchstens 7 % reduziert werden. Dabei bleiben die anderen interessierenden Werte wie Hydroperoxidumsatz, Selektivität hinsichtlich der Epoxidfolgeprodukte und die Propylenoxidselektivität in den oben genannten bevorzugten Bereichen.

Im allgemeinen kann das poröse oxidische Material des Titanzeolithen als solches als Katalysator zur Epoxidierung eingesetzt werden, wie dies auch in der bereits oben genannten WO 02/28774 A2 im Falle der Umsetzung von Olefinen mit mindestens einer weiteren funktionellen Gruppe oder in dem ebenfalls bereits oben genannten Artikel von Wu et al. im Falle der Umsetzung von Cyclohexen beschrieben ist.

Gemäß einer bevorzugten Ausführungsform des erfindungsgemäßen Verfahrens werden das poröse oxidische Material, die Aktivkomponente des Zeolith-Katalysators, nicht direkt nach der Calcinierung als Katalysator verwendet, sondern in einem zusätzlichen Verarbeitungsschritt aus dem porösen oxidischen Material Katalysatorformkörper hergestellt.

Demgemäß betrifft die vorliegende Erfindung auch ein Verfahren, wie oben beschrieben, dass dadurch gekennzeichnet ist, dass die Herstellung des Katalysators gemäß (i) mindestens die Schritte (a) und (b) umfasst:

(a) Herstellung des mindestens einen porösen oxidischen Materials;

(b) Herstellung eines Formkörpers unter Verwendung des gemäß (a) erhaltenen porösen oxidischen Materials.

Die Herstellung der Katalysatorformkörper kann hierbei generell gemäß sämtlicher geeigneter Verfahren durchgeführt werden. Was den spezifischen Schritt der Herstellung eines Formkörpers anbelangt, so sei auf die WO 98/55229 und die DE 102 32 406.9 verwiesen, deren diesbezüglicher Inhalt durch Bezugnahme in den Kontext der vorliegenden Anmeldung einbezogen wird.

Bevorzugt wird zu dem zeolithischen Material, das aus der Mutterlauge abgetrennt und gegebenenfalls mindestens einer der oben angegebenen weiteren Behandlungen wie Waschen, Trocknen, Calcinieren, Inkontaktbringen mit einer Wasser enthaltenden Zusammensetzung oder Behandeln mit Wasserstoffperoxidlösung unterworfen wurde, mit mindestens einem Bindemittel versetzt. Weitere Zusatzstoffe wie beispielsweise Mischungen aus Wasser und mindestens einem Alkohol oder mindestens eine viskositätssteigernde organische Verbindung oder mindestens eine porenbildende Verbindung, wie sie aus dem Stande der Technik bekannt sind, können ebenfalls zugegeben werden.

Als Bindemittel kann im Wesentlichen jede Verbindung eingesetzt werden, die die Kohäsion zwischen den Teilchen des zeolithischen Materials erhöht. Bevorzugte Bindemittel sind solche aus der Gruppe bestehend aus hydratisiertem Kieselgel, Kieselsäure, Kieselgel, Tetraalkoxysilikaten, Tetraalkoxytitanaten, Tetraalkoxyzirkonaten und Mischungen aus zwei oder mehr davon. Besonders bevorzugt sind Tetramethoxysilikat, Tetraethoxysilikat, Tetrapropoxysilikat, Tetrabutoxysilikat oder Kieselsol. Besonders bevorzugt sind Tetramethoxysilikat, Tetraethoxysilikat und Kieselsol, wobei insbesondere bevorzugt Kieselsol ist.

Weitere Bindemittel sind in der WO 98/55229 und der DE 102 32 406.9 beschrieben, deren diesbezüglicher Inhalt durch Bezugnahme in den Kontext der vorliegenden Anmeldung einbezogen wird.

Die genannten Bindemittel sind entweder allein oder als Mischungen aus zwei oder mehr davon einsetzbar. Weitere Bindemittel wie Oxide des Siliciums, Bors, Phosphors, Zirkoniums und/oder Titans können zusätzlich verwendet werden.

Im Rahmen der erfindungsgemäßen Herstellung des Formkörpers werden im allgemeinen bis zu 80 Gew.-%, bevorzugt 10 bis 75 Gew.-% und besonders bevorzugt 20 bis 40 Gew.-% Bindemittel, bezogen auf das Gesamtgewicht des Formkörpers, eingesetzt.

Gemäß einer weiter bevorzugten Ausführungsform des erfindungsgemäßen Verfahrens wird dem zeolithischen Material mindestens ein Porenbildner zugefügt. Bevorzugt werden hierbei Polymere und weiter bevorzugt Polymere, die in Wasser oder wässrigen Lösungsmitteln dispergierbar, emulgierbar oder suspendierbar sind, verwendet. Dieses mindestens eine Polymer wird bevorzugt ausgewählt aus der Gruppe bestehend aus Vinylpolymeren wie beispielsweise Polystyrol, Polyacrylaten, Polymethacrylaten, Polyolefinen, Polyamiden und Polyestern. Diese porenbildenden Polymere werden nach der Herstellung der Formkörper durch Calcinieren bei geeigneten Temperaturen aus dem Formkörper entfernt. Werden Polymere als Porenbildner zugegeben, so werden diese in einem Anteil von im allgemeinen 5 bis 50 Gew.-%, bevorzugt 7 bis 35 Gew.-% und besonders bevorzugt von 10 bis 20 Gew.-%, jeweils bezogen auf das Gesamtgewicht der anorganischen Anteile aus Bindemittel und Zeolith, zugegeben.

Weiter bevorzugt wird mindestens ein Verstrangungshilfsmittel zugegeben. Als Verstrangungshilfsmittel kann im wesentliches jede Verbindung eingesetzt werden, die zur Verbesserung der Misch-, Knet- oder Fließeigenschaften führt. Bevorzugt sind organische hydrophile Polymere wie beispielsweise Cellulose, Cellulosederivate wie beispielsweise Alkylcellulosen, Stärke, Polyacrylate, Polymethacrylate, Polyvinylalkohol, Polyvinylpyrrolidon, Polyisobuten oder Polytetrahydrofuran. Erfindungsgemäß werden diese Verbindungen auch deshalb eingesetzt, weil sie die mechanische Stabilität der Formkörper, bevorzugt beim Formen und Trocknen und bei der anschließenden Verwendung als Katalysatorformkörper, erhöhen. Diese Verbindungen werden aus dem Formkörper durch Calcinieren bei geeigneten Temperaturen entfernt.

Weitere Zusatzstoffe sind in EP 0 389 041 A, EP 0 200 260 A und in WO 95/19222 beschrieben, deren diesbezüglicher Inhalt durch Bezugnahme in den Kontext der vorliegenden Anmeldung einbezogen wird.

Gemäß einer bevorzugten Ausführungsform des erfindungsgemäßen Verfahrens wird nach der Zugabe des mindestens einen Bindemittels zum zeolithischen Material die mindestens eine organische viskositätssteigernde Verbindung zugegeben und die erhaltene Masse im Bereich von 10 bis 180 min. in einer Knetvorrichtung oder einem Extruder homogenisiert.

Diese Homogenisierung erfolgt bei Temperaturen, die im Allgemeinen etwa 10 °C unter dem Siedepunkt des Verstrangungshilfsmittels liegen. Dabei wird im Allgemeinen ein Druck angewandt, der in etwa bei Umgebungsdruck oder bei leichtem Überdruck liegt.

Gemäß einer weiteren bevorzugten Ausführungsform des erfindungsgemäßen Verfahrens wird während des Knetvorganges zum zeolithischen Material zunächst die mindestens eine porenbildende Verbindung und anschließend das mindestens eine Bindemittel zugegeben, woraufhin in einem oder auch mehreren Schritten die mindestens eine Wasser enthaltende Zusammensetzung, bevorzugt Wasser, zugegeben wird. Gemäß einer weiter bevorzugten Ausführungsform wird zunächst während des Knetens die mindestens eine porenbildende Verbindung und anschließend ein Teil des mindestens einen Bindemittels zugegeben, woraufhin in einem oder auch mehreren Schritten ein Teil der mindestens einen Wasser enthaltenden Zusammensetzung, bevorzugt Wasser, zugegeben wird. Im Anschluss daran wird der restliche Teil des Bindemittels und daraufhin in einem oder mehreren Schritten der Rest der mindestens einen Wasser enthaltenden Zusammensetzung, bevorzugt Wasser, zugegeben.

Bevorzugt werden dem zeolithischen Material Kieselsol und/oder eine Polystyroldispersion und/oder Cellulose und/oder ein Cellulosederivat wie beispielsweise Alkylcellulose und/oder Polyethylenoxid und/oder Wasser zugegeben. Beispielsweise bevorzugt wird das zeolithische Material mit Kieselsol, einer Polystyroldispersion, Methylcellulose und Wasser versetzt und dann in einer geeigneten Vorrichtung durch Kneten homogenisiert.

Demgemäß beschreibt die vorliegende Erfindung auch ein Verfahren, wie oben beschrieben, das dadurch gekennzeichnet ist, dass die Herstellung des Formkörpers gemäß (b) mindestens den Schritt (aa) umfasst:

(aa) Kneten des gemäß (a) erhaltenen porösen oxidischen Materials unter Zugabe mindestens eines Bindemittels oder mindestens eines Verstrangungshilfsmittels oder mindestens eines Porenbildners oder einer Wasser enthaltenden Zusammensetzung oder eines Gemischs aus zwei oder mehr davon.

Gemäß einer weiter bevorzugten Ausführungsform wird nach dem Kneten der Masse, wie oben beschrieben, die derart geknetete Masse zu einem Formkörper verformt. Dies kann generell gemäß sämtlicher geeigneter Verfahren erfolgen. Bevorzugt werden im erfindungsgemäßen Verfahren die Formkörper mittels eines Extruders hergestellt.

Wiederum bevorzugt werden hierbei Extrudate hergestellt, die einen Durchmesser im Bereich von 1 bis 10 mm, weiter bevorzugt von 1 bis 5 mm und insbesondere bevorzugt von 1 bis 2 mm aufweisen.

Die Verformung kann bei Umgebungsdruck oder bei im Vergleich zum Umgebungsdruck erhöhtem Druck im Bereich von im allgemeinen 1 bis 700 bar erfolgen. Weiterhin kann die Verformung bei Umgebungstemperatur oder bei im Vergleich zur Umgebungstemperatur erhöhter Temperatur im Bereich von im allgemeinen 20 bis 300 °C erfolgen. Weiter kann die Verformung in kontrollierter Atmosphäre durchgeführt werden, wobei generell inerte Gasatmosphären, reduzierende oder oxidierende Atmosphären möglich sind.

Die Abtrennung der einzelnen Formkörper aus dem aus dem Extruder austretenden Formmassenstrang kann generell gemäß sämtlicher möglicher Verfahren erfolgen. Besonders bevorzugt wird der pastöse Formmassenstrang im Extruder dadurch abgetrennt, dass zum Zertrennen die pastöse Formmasse mit mindestens einem Strom, enthaltend mindestens ein fluides Medium, in Kontakt gebracht wird. Weiter bevorzugt ist das fluide Medium ein Gas oder eine Flüssigkeit, insbesondere bevorzugt im wesentlichen Luft. Ebenfalls weiter bevorzugt wird der Strang der pastösen Formmasse periodisch zertrennt. Durch dieses Verfahren ist es möglich, im Vergleich zu den mechanischen Abtrennverfahren des Standes der Technik Formkörper herzustellen, die eine höhere Schüttdichte aufweisen. Dies wirkt sich insbesondere bei der Verwendung in Festbettreaktoren vorteilhaft aus.

Demgemäß beschreibt die vorliegende Erfindung auch ein Verfahren, wie oben beschrieben, das dadurch gekennzeichnet ist, dass die Herstellung des Formkörpers gemäß (b) mindestens die Schritte (aa) und (bb) umfasst:

(aa) Kneten des gemäß (a) erhaltenen porösen oxidischen Materials unter Zugabe mindestens eines Bindemittels oder mindestens eines Verstrangungshilfsmittels oder mindestens eines Porenbildners oder einer Wasser enthaltenden Zusammensetzung oder eines Gemischs aus zwei oder mehr davon;
(bb) Verformen der gemäß (aa) erhaltenen gekneteten Mischung unter Erhalt mindestens eines Formkörpers.

Ganz allgemein betrifft die vorliegende Erfindung daher auch einen Katalysatorformkörper, als katalytisch aktives Material mindestens enthaltend ein poröses oxidisches Material, **dadurch gekennzeichnet, dass** das poröse oxidische Material ein Zeolith mit röntgenographischer Zuordnung zum MWW-Typ ist.

Neben dem Zeolith mit röntgenographischer Zuordnung zum MWW-Typ kann der erfindungsgemäße Formkörper beispielsweise mindestens einen der wie oben beschrieben Zusatzstoffe, die bei der Herstellung des Formkörpers eingesetzt werden können, enthalten.

Weiter bevorzugt werden die Formkörper daraufhin bei Temperaturen im Bereich von 30 bis 140 °C, bevorzugt von 60 bis 135 °C und besonders bevorzugt von 90 bis 130 °C getrocknet, wobei die Trocknungszeiträume im allgemeinen im Bereich von 1 bis 20 h, bevorzugt im Bereich von 2 bis 10 h und besonders bevorzugt im Bereich von 3 bis 5 h liegen. Dabei werden Aufheizraten von im allgemeinen 0,5 bis 5 °C/min, bevorzugt von 1 bis 4 °C/min und besonders bevorzugt von 1,5 bis 3 °C/min gewählt.

Demgemäß beschreibt die vorliegende Erfindung auch ein Verfahren, wie oben beschrieben, das dadurch gekennzeichnet ist, dass die Herstellung des Formkörpers gemäß (b) mindestens die Schritte (aa) bis (cc) umfasst:

(aa) Kneten des gemäß (a) erhaltenen porösen oxidischen Materials unter Zugabe mindestens eines Bindemittels oder mindestens eines Verstrangungshilfsmittels oder mindestens eines Porenbildners oder einer Wasser enthaltenden Zusammensetzung oder eines Gemischs aus zwei oder mehr davon;
(bb) Verformen der gemäß (aa) erhaltenen gekneteten Mischung unter Erhalt mindestens eines Formkörpers;

(cc) Trocknen des gemäß (bb) erhaltenen Formkörpers.

Weiter bevorzugt werden die getrockneten Formkörper daraufhin bei Temperaturen im Bereich von 400 bis 800 °C, bevorzugt von 425 bis 600 °C und besonders bevorzugt von 450 bis 500 °C calciniert, wobei die Calcinierungszeiträume im allgemeinen im Bereich von 1 bis 20 h, bevorzugt im Bereich von 2 bis 10 h und besonders bevorzugt im Bereich von 3 bis 7 h liegen. Dabei werden Aufheizraten von im allgemeinen 0,25 bis 2 °C/min, bevorzugt von 0,5 bis 1,5 °C/min und besonders bevorzugt von 0,75 bis 1,25 °C/min gewählt. Ganz besonders bevorzugt wird der getrocknete Formkörper unter Luft und/oder Magerluft calciniert.

Demgemäß betrifft die vorliegende Erfindung auch ein Verfahren, wie oben beschrieben, das dadurch gekennzeichnet ist, dass die Herstellung des Formkörpers gemäß (b) mindestens die Schritte (aa) bis (dd) umfasst:

(aa) Kneten des gemäß (a) erhaltenen porösen oxidischen Materials unter Zugabe mindestens eines Bindemittels oder mindestens eines Verstrangungshilfsmittels oder mindestens eines Porenbildners oder einer Wasser enthaltenden Zusammensetzung oder eines Gemischs aus zwei oder mehr davon;
(bb) Verformen der gemäß (aa) erhaltenen gekneteten Mischung unter Erhalt mindestens eines Formkörpers;
(cc) Trocknen des gemäß (bb) erhaltenen Formkörpers;
(dd) Calcinieren des gemäß (cc) erhaltenen getrockneten Formkörpers.

Demgemäß beschreibt die vorliegende Erfindung auch einen Katalysatorformkörper, wie oben beschrieben, erhältlich gemäß einem Verfahren, umfassend die Stufe (aa) oder die Stufen (aa) und (bb) oder die Stufen (aa), (bb) und (cc) oder die Stufen (aa), (bb), (cc) und (dd).

Vor oder nach dem Trocknen und/oder Calcinieren der gemäß (bb) erhaltenen Formkörper können diese, wie oben hinsichtlich des zeolithischen Materials beschrieben, mit einer Wasser enthaltenden Zusammensetzung in Kontakt gebracht werden. Werden die Formkörper nach dem Trocknen und/oder Calcinieren mit der Wasser enthaltenden Zusammensetzung in Kontakt gebracht, so schließt sich bevorzugt ein nochmaliges Trocknen und/oder Calcinieren an, das gemäß den unter (cc) und/oder (dd) beschriebenen Verfahrensführungen durchgeführt wird.

Demgemäß beschreibt die vorliegende Erfindung auch einen Katalysatorformkörper, wie oben beschrieben, erhältlich gemäß einen Verfahren, umfassend die Stufe (aa) oder die Stufen (aa) und (bb) oder die Stufen (aa), (bb) und (cc) oder die Stufen (aa), (bb), (cc) und (dd), **dadurch gekennzeichnet, dass** nach Stufe (bb) oder Stufe (cc) oder Stufe (dd) der Formkörper mit einer Wasser enthaltenden Zusammensetzung in Kontakt gebracht wird.

Gemäß des erfindungsgemäßen Verfahrens werden bevorzugt Formkörper erhalten, die Poren aufweisen, deren Volumina im Allgemeinen im Bereich von 0,5 bis 2,0 ml/g, bevorzugt im Bereich von 0,7 bis 1,4 ml/g und besonders bevorzugt im Bereich von 0,9 bis 1,3 ml/g liegen. Die Porenvolumina verstehen sich hierbei als durch Quecksilberporosimetrie nach DIN 66133 bestimmte Größen.

Bei den bevorzugt erhaltenen Formkörpern liegt der Durchmesser von etwa 80 % der Poren im Bereich von 4 nm bis 1 $\mu$m Hinsichtlich der Poren existiert hierbei eine bimodale Verteilung, wobei das Maximum der Porendurchmesser bei etwa 60 nm und das Minimum bei etwa 20 nm liegt.

Bei bevorzugt erhaltenen Formkörpern in der Form von Strängen mit einem Durchmesser von 1,5 mm liegt die Seitendruckfestigkeit, bestimmt mit einem Härtemessgerät der Firma Zwick, Ulm, vom Typ Z2.5/TS1S bei einer Vorkraftgeschwindigkeit von 10 mm/min und einer Prüfkraftgeschwindigkeit von 1,6 mm/min, im Bereich von 2 bis 9 N. Details zu diesem Gerät sind dem entsprechenden "Technischen Handbuch 441801" zu entnehmen.

Demgemäß beschreibt die vorliegende Erfindung auch ein Verfahren, wie oben beschrieben, das dadurch gekennzeichnet ist, dass der Durchmesser von 80 % der bimodal verteilten Poren des Formkörpers im Bereich von 4 nm bis 1 $\mu$m liegt.

Ebenso betrifft die vorliegende Erfindung daher auch einen Formkörper, wie oben beschrieben, der dadurch gekennzeichnet ist, dass der Durchmesser von 80 % der bimodal verteilten Poren des Formkörpers im Bereich von 4 nm bis 1 $\mu$m liegt.

Im erfindungsgemäßen Verfahren wird zur Umsetzung des Propens mindestens ein Hydroperoxid eingesetzt. Im Rahmen der vorliegenden Anmeldung wird unter dem Begriff "Hydroperoxid" eine Verbindung der allgemeinen Formel ROOH verstanden. Details bezüglich der Hydroperoxidherstellung und von unter anderem im Rahmen des erfindungsgemäßen Verfahrens einsetzbaren Hydroperoxiden sind der DE 198 35 907 A zu entnehmen, deren diesbezüglicher Inhalt in den Kontext der vorliegenden Anmeldung aufgenommen wird. Beispiele für erfindungsgemäß einsetzbare Hydroperoxide sind unter anderem tert-Butylhydroperoxid, Ethylbenzolhydroperoxid, tert-Amylhydroperoxid, Cumenhy-droperoxid, Cyclohexylhydroperoxid, Methylcyclohexylhydroperoxid, Tetrahydronaphthalinhydroperoxid, Isobutylben-zolhydroperoxid, Ethylnaphthalinhydroperoxid, Persäuren wie beispielsweise Peressigsäure oder Wasserstoffperoxid. Auch Gemische aus zwei oder mehr Hydroperoxiden sind erfindungsgemäß einsetzbar. Bevorzugt wird im Rahmen der vorliegenden Erfindung Wasserstoffperoxid als Hydroperoxid eingesetzt, wobei weiter bevorzugt eine wässrige Wasserstoffperoxidlösung eingesetzt wird.

Demgemäß beschreibt die vorliegende Erfindung auch ein Verfahren, wie oben beschrieben, das dadurch gekennzeichnet ist, dass das Hydroperoxid Wasserstoffperoxid ist.

Als Lösungsmittel zur Umsetzung von Propen zu Propylenoxid wird erfindungsgemäß mindestens ein Nitril oder ein mindestens ein Nitril enthaltendes Lösungsmittelgemisch eingesetzt. Beispiele für solche Nitrile sind etwa Acetonitril, Propionitril oder Benzonitril.

Wird im erfindungsgemäßen Verfahren ein mindestens ein Nitril enthaltendes Lösungsmittelgemisch eingesetzt, so können neben dem mindestens einen Nitril sämtliche zur Umsetzung von Propen zu Propylenoxid geeigneten Lösungsmittel eingesetzt werden. Unter anderem sind hierbei zu nennen:

- Wasser;
- Alkohole, bevorzugt Alkohole mit weniger als 6 Kohlenstoffatomen wie beispielsweise Methanol, Ethanol, Propanole, Butanole und Pentanole;
- Diole oder Polyole, bevorzugt solche mit weniger als 6 Kohlenstoffatomen;
- Ester wie beispielsweise Methylacetat oder Butyrolacton;
- Amide wie beispielsweise Dimethylformamid, Dimethyacetamid oder N-Methylpyro lidon;
- Ketone wie beispielsweise Aceton;
- Gemische aus zwei oder mehr der vorgenannten Verbindungen.

Besonders bevorzugt wird erfindungsgemäß Acetonitril oder ein Gemisch aus Wasser und Acetonitril als Lösungsmittel eingesetzt.

Demgemäß beschreibt die vorliegende Erfindung auch ein Verfahren, wie oben beschrieben, das dadurch gekennzeichnet ist, dass das mindestens eine Nitril Acetonitril ist.

Ebenso beschreibt die vorliegende Erfindung auch ein Verfahren, wie oben beschrieben, das dadurch gekennzeichnet ist, dass das mindestens ein Nitril enthaltende Lösungsmittelgemisch ein Gemisch aus Wasser und Acetonitril ist.

Weiter betrifft die vorliegende Erfindung daher auch ein Verfahren, wie oben beschrieben, das dadurch gekennzeichnet ist, dass das Hydroperoxid Wasserstoffperoxid und das mindestens eine Nitril Acetonitril ist.

Die Umsetzung gemäß (ii) kann generell gemäß sämtlicher geeigneter Verfahrensführungen erfolgen. So sind beispielsweise die Fahrweisen in einem Batchreaktor oder in mindestens einem kontinuierlich betriebenen Reaktor möglich. Insbesondere bei Verwendung der oben beschriebenen Katalysator-Formkörper ist die kontinuierliche Fahrweise bevorzugt.

Demgemäß beschreibt die vorliegende Erfindung auch ein Verfahren, wie oben beschrieben, das dadurch gekennzeichnet ist, dass die Umsetzung gemäß (ii) in mindestens einem kontinuierlich betriebenen Reaktor erfolgt.

Gemäß einer bevorzugten Ausführungsform erfolgt die Umsetzung in einem einzigen, kontinuierlich betriebenen Reaktor. Im Rahmen dieser Ausführungsform erfolgt die Umsetzung bevorzugt bei Temperaturen im Bereich von 20 bis 80 °C, weiter bevorzugt im Bereich von 25 bis 70 °C und besonders bevorzugt im Bereich von 30 bis 65 °C. Die Drücke im Reaktor werden hierbei im Bereich von 15 bis 45 bar, bevorzugt von 20 bis 40 bar und besonders bevorzugt von 25 bis 35 bar gewählt. In besonders bevorzugten Ausführungsformen des erfindungsgemäßen Verfahrens wird die Umsetzung bei Temperaturen und Drücken durchgeführt, bei denen das Reaktionsgemisch flüssig und einphasig ist.

Demgemäß beschreibt die vorliegende Erfindung auch ein Verfahren, wie oben beschrieben, das dadurch gekennzeichnet ist, dass die Umsetzung gemäß (ii) bei Temperaturen im Bereich von 20 bis 80°C und bei Drücken im Bereich von 15 bis 45 bar durchgeführt wird.

Daher betrifft die vorliegende Erfindung auch ein Verfahren, wie oben beschrieben, das dadurch gekennzeichnet ist, dass die Umsetzung gemäß (ii) in mindestens einem kontinuierlich betriebenen Reaktor bei Temperaturen im Bereich von 20 bis 80 °C und bei Drücken im Bereich von 15 bis 45 bar durchgeführt wird.

Die Standzeit des Katalysators im Reaktor liegt hierbei im allgemeinen bei mindestens 24 h, bevorzugt im Bereich von bis zu 200 h, weiter bevorzugt im Bereich von bis zu 500 h und besonders bevorzugt im Bereich von bis zu 1000 h.

Der im Verfahren verwendete Katalysator kann nach der Reaktion entweder im Reaktor oder außerhalb des Reaktors oder sowohl im Reaktor als auch außerhalb des Reaktors gemäß einem oder mehreren geeigneten Verfahren regeneriert werden. Gemäß einem bevorzugten Verfahren wird der Katalysator in dem Reaktor, in dem die Umsetzung des Propens erfolgt, mittels einer thermischen Behandlung des Katalysators in Gegenwart eines Gasstroms bei Temperaturen oberhalb von 120°C, vorzugsweise oberhalb von 350°C und insbesondere bei 400°C bis 650°C regeneriert, wobei während der thermischen Behandlung die massenbezogene Verweilzeit des Gasstroms über dem Katalysator mehr als 2 Stunden beträgt und bevorzugt im Bereich von 3 bis 10 Stunden und insbesondere bevorzugt im Bereich von 4 bis 6 Stunden liegt. Das Regeneriergas enthält hierbei im Allgemeinen weniger als 20 Vol.-%, bevorzugt 0,1 bis 10 Vol.-%, insbesondere 0,1 bis 5 Vol.-% und noch weiter bevorzugt 0,1 bis 2 Vol.-% Sauerstoff. Vorzugsweise wird ein Gemisch aus Luft und entsprechenden Volumina Stickstoff eingesetzt. Der erfindungsgemäß verwendete Begriff "massenbezogene Verweilzeit" bezeichnet dabei das Verhältnis der Katalysator-Masse ($M_{Kat}$), dividiert durch den Massenstrom ($M_{Gase}$) des bei der Regenerierung verwendeten Gases. Dabei wird im allgemeinen so gefahren, dass der Druckverlust über dem Reaktor nicht mehr als 4 bar, vorzugsweise nicht mehr als 3 bar und insbesondere nicht mehr als 2,5 bar beträgt.

Gemäß einer ebenfalls bevorzugten Ausführungsform des erfindungsgemäßen Verfahrens kann die Umsetzung des Propens in zwei oder mehr Stufen erfolgen. Eine zweistufige Umsetzung findet beispielsweise folgendermaßen statt:

(A) Umsetzung von Propen mit einem Hydroperoxid, bevorzugt Wasserstoffperoxid, in mindestens einem Nitril als Lösungsmittel oder in einem mindestens ein Nitril enthaltenden Lösungsmittelgemisch in Anwesenheit des Katalysators gemäß (i) unter Erhalt einer Mischung, enthaltend Propylenoxid und nicht umgesetztes Hydroperoxid, bevorzugt nicht umgesetztes Wasserstoffperoxid;

(B) Abtrennung des nicht-umgesetzten Hydroperoxids, bevorzugt Wasserstoffperoxid, aus der aus (A) resultierenden Mischung;

(C) Umsetzung des abgetrennten Hydroperoxids, bevorzugt Wasserstoffperoxid, aus (B) mit Propen.

Ebenso sind auch mehr als zwei Umsetzungsstufen und mehr als eine Abtrennstufe möglich. Hinsichtlich der mehrstufigen Umsetzung sei auf die DE 198 35 907 A verwiesen, die diesbezüglich durch Bezugnahme in den Kontext der vorliegenden Anmeldung einbezogen wird.

Bevorzugt erfolgt hierbei die Umsetzung in vier, weiter bevorzugt in drei und insbesondere bevorzugt in zwei Stufen mit einer zwischengeschalteten Abtrennstufe, wie oben beschrieben. Ganz besonders bevorzugt werden die Umsetzungen gemäß (A) und (C) in jeweils einem Festbettreaktor und besonders bevorzugt in jeweils einem Festbettrohrreaktor durchgeführt. Insbesondere bevorzugt werden die Umsetzung gemäß (A) in einem isothermen Festbettreaktor und die Umsetzung gemäß (C) in einem adiabatischen Festbettreaktor durchgeführt.

Im Rahmen dieser Ausführungsform erfolgt die Umsetzung gemäß (A) bevorzugt bei Temperaturen im Bereich von 20 bis 80 °C, weiter bevorzugt im Bereich von 25 bis 70 °C und besonders bevorzugt im Bereich von 30 bis 65 °C. Die Drücke im Reaktor werden hierbei im Bereich von 15 bis 45 bar, bevorzugt von 20 bis 40 bar und besonders bevorzugt von 25 bis 35 bar gewählt.

Die Umsetzung gemäß (C) erfolgt bevorzugt bei Temperaturen im Bereich von 20 bis 80 °C, weiter bevorzugt im Bereich von 25 bis 70 °C und besonders bevorzugt im Bereich von 30 bis 65 °C. Die Drücke im Reaktor werden hierbei im Bereich von 15 bis 45 bar, bevorzugt von 20 bis 40 bar und besonders bevorzugt von 25 bis 35 bar gewählt.

Die Abtrennung des eingesetzten Hydroperoxids gemäß (B) kann gemäß sämtlicher geeigneter Verfahren erfolgen. Die Abtrennung des bevorzugt eingesetzten Wasserstoffperoxids erfolgt bevorzugt destillativ, wobei eine oder mehrere, bevorzugt eine Destillationskolonne eingesetzt wird.

Im Falle, dass zwei oder mehr Reaktoren zur Umsetzung eingesetzt werden, erfolgt die Reaktorverschaltung bevorzugt derart, dass im ersten Reaktor ein Hydroperoxidumsatz im Bereich von 85 bis 95 % eingestellt wird und der Restumsatz in dem mindestens einen weiteren Reaktor erzielt wird.

Der in dem jeweiligen Reaktor verwendete Katalysator kann wie oben beschrieben regeneriert werden, wobei der Katalysator eines Reaktors auf die gleiche oder eine unterschiedliche Art und Weise wie der Katalysator eines anderen Reaktors regeneriert werden kann.

Gemäß einer weiteren Ausführungsform des erfindungsgemäßen Verfahrens kann die Umsetzung des Propens mit dem Hydroperoxid auch in einem oder mehreren Reaktoren, wie oben beschrieben, durchgeführt werden, wobei mindestens einer der Reaktoren in Suspensionsfahrweise betrieben wird.

Ganz allgemein betrifft die vorliegende Erfindung die Verwendung eines Titanzeolithen mit röntgenographischer Zuordnung zum MWW-Typ zur Epoxidierung von Propen mit einem Hydroperoxid, vor allem die Verwendung als Katalysator zur Epoxidierung von Propen mit einem Hydroperoxid, bevorzugt mit Wasserstoffperoxid.

Weiterhin beschreibt die vorliegende Erfindung die Verwendung eines Titanzeolithen mit röntgenographischer Zuordnung zum MWW-Typ zur Epoxidierung von Propen mit Wasserstoffperoxid in Acetonitril als Lösungsmittel.

Das erfindungsgemäße Verfahren kann auch in mindestens einem Reaktor unter Verwendung von mindestens zwei voneinander verschiedenen Zeolith-Katalysatoren durchgeführt werden, wobei mindestens zwei der voneinander verschiedenen Zeolith-Katalysatoren räumlich voneinander getrennt eingesetzt werden. Mindestens einer der Zeolithkatalysatoren ist hierbei ein Zeolithkatalysator, bevorzugt ein Titanzeolithkatalysator vom Kristallstrukturtyp MWW. Wie oben beschrieben, können die Zeolithkatalysatoren als zeolithisches Material an sich oder als Formkörper eingesetzt werden, wobei die Unterschiede zwischen den zeolithischen Materialien oder zwischen den Formkörpern bestehen können.

Unterschiede hinsichtlich der zeolithischen Materialien sind beispielsweise

- der Titangehalt des zeolithischen Materials;
- der Gehalt an anderen chemischen Elementen neben Titan;

- die Porosität des Zeolithen, wobei sich hinsichtlich der Porosität beispielsweise die Geometrien der Poren der voneinander verschiedenen Zeolith-Katalysatoren unterscheiden und diese demgemäß beispielsweise unterschiedliche Porenvolumina, unterschiedliche Porendurchmesser oder unterschiedliche Oberflächen der Poren aufweisen; ebenso können sich die Zeolithen in der Porenverteilung unterscheiden.
- die Kristallstruktur des zeolithischen Materials;
- die Oberflächenmodifizierung des zeolithischen Materials;
- die Acidität des zeolithischen Materials.

Unterschiede hinsichtlich der Formkörper sind beispielsweise

- die Geometrie der Katalysatorformkörper;
- die Porosität der Formkörper;
- die mechanische Festigkeit der Formkörper;
- der Bindergehalt der Katalysatorformkörper;
- der Typ des zur Herstellung der Katalysatorformkörper verwendeten Bindermaterials;
- der Gehalt der Katalysatorformkörper an katalytisch aktivem zeolithischen Material;
- der Kohlenstoffgehalt der Formkörper.

Der Begriff "voneinander verschiedene Zeolith-Katalysatoren" umfasst im Rahmen der vorliegenden Erfindung auch zwei voneinander verschiedene Katalysatormischungen, wobei eine Mischung mindestens zwei unterschiedliche Formkörper oder mindestens zwei unterschiedliche zeolithische Materialien als solche oder mindestens einen Formkörper und mindestens ein zeolithisches Material als solches enthalten kann. Voneinander verschiedene Katalysatormischungen bezeichnen somit oben beschriebene Mischungen, die sich

- entweder in mindestens einem der oben beispielhaft beschriebenen Unterscheidungsmerkmale bezüglich des zeolithischen Materials oder der Formkörper
- oder im Mischungsverhältnis der in der Mischung enthaltenen Komponenten
- oder sowohl in mindestens einem der oben beispielhaft beschriebenen Unterscheidungsmerkmale bezüglich des zeolithischen Materials oder der Formkörper als auch im Mischungsverhältnis der in der Mischung enthaltenen Komponenten

unterscheiden.

Der Begriff "räumlich voneinander getrennt", wie er im Rahmen der vorliegenden Erfindung verwendet wird, bezeichnet Ausführungsformen, bei denen die Vorrichtung, in der die Umsetzung durchgeführt wird, mindestens zwei Kompartimente aufweist, wobei eines der Kompartimente einen Zeolith-Katalysator enthält und mindestens ein anderes Kompartiment mindestens einen weiteren Zeolith-Katalysator enthält, der sich von dem im ersten Kompartiment enthaltenen Zeolith-Katalysatoren unterscheidet.

Eine solche Kompartimentierung kann beispielsweise in einem einzigen Reaktor realisiert werden, wobei wiederum verschiedene Ausführungsformen der Kompartimentierung möglich sind.

Gemäß einer besonders bevorzugten Ausführungsform wird diese Kompartimentierung beispielsweise durch eine strukturierte Schüttung verschiedener Katalysatoren erreicht. Eine erste Zone des Reaktors wird dabei durch Schüttung mit einem ersten Zeolith-Katalysator ausgestattet, wodurch das erste Katalysator-Kompartiment hergestellt wird. Anschließend wird eine zweite Zone des Reaktors durch Aufschütten des zweiten, vom ersten verschiedenen Zeolith-Katalysator auf das erste Kompartiment unter Ausbildung des zweiten Kompartiments hergestellt. Ebenso können auch ein drittes oder weitere Kompartimente hinzugefügt werden, wobei im dritten oder einem weiteren Kompartiment jeweils einer der beiden ersten oder ein von den ersten beiden Katalysatoren verschiedener Zeolith-Katalysator verwendet werden kann. Diese Art der Herstellung wird im Rahmen der vorliegenden Erfindung als "strukturierte Schüttung" bezeichnet.

Diese strukturierte Schüttung bietet unter anderem im Vergleich zu herkömmlichen Verfahren, bei denen ein Reaktor mit nur einem einzigen Zeolith-Katalysator ausgestattet wird, den Vorteil, dass durch die gezielte Auswahl der Katalysatoren, die in verschiedenen Reaktorzonen eingesetzt werden, beispielsweise der Reaktionsumsatz positiv beeinflussen lässt. Beispielsweise bei kontinuierlicher Reaktionsführung, bei denen die Reaktionspartner Propen und Hydroperoxid durch den Reaktor geleitet werden und dabei die verschiedenen Reaktorzonen mit den unterschiedlichen Zeolith-Katalysatoren passieren, können die einzelnen Katalysatoren dem Reaktionsfortschritt angepasst werden.

So ist es beispielsweise möglich, in einer ersten Zone des Reaktors, in der die Konzentration der nicht umgesetzten Reaktionspartner hoch ist, den Zeolith-Katalysator so zu wählen, dass der Umsatz beispielsweise bei einer exothermen Reaktion gerade so hoch ist, dass die entstehende Wärme noch abgeführt werden kann. In einer nächsten Reaktorzone, in der die Konzentration der Reaktionspartner geringer ist, kann dann ein Zeolith-Katalysator eingesetzt werden, der beispielsweise einen höheren Umsatz gewährleistet, der also hinsichtlich der Reaktion aktiver ist. Die beim Durchgang der Reaktionspartner Hydroperoxid und Propen durch den Reaktor entstehende Inhomogenität in deren Konzentrationen und die dadurch resultierende Inhomogenität des Reaktionsgemisches, enthaltend die Reaktionspartner Hydroperoxid und Propen und das daraus entstehende Reaktionsprodukt oder die daraus entstehenden Reaktionsprodukte, können demgemäß durch eine entsprechende Wahl von unterschiedlichen Zeolith-Katalysatoren und damit durch eine Inhomogenität von beispielsweise der Katalysatoraktivität über den Reaktor hinweg kompensiert werden.

Beispielsweise ist es möglich, dass bei der Umsetzung von Hydroperoxid mit Propen beispielsweise Produkte entstehen, die in der Lage sind, wiederum mit entweder Hydroperoxid oder Propen oder sowohl mit Hydroperoxid und Propen zu einem unerwünschten Folgeprodukt weiterzureagieren. Beim Durchgang des Reaktionsgutes durch den Reaktor werden in diesem Fall die Konzentration an erwünschtem Produkt und damit die Wahrscheinlichkeit, dass sich unerwünschtes Folgeprodukt bildet, immer größer. Dementsprechend können beispielsweise in einer ersten Reaktorzone ein erster Zeolith-Katalysator eingesetzt werden und in einer zweiten Reaktorzone ein anderer Zeolith-Katalysator eingesetzt werden, der die Umsetzung von Hydroperoxid und organischer Verbindung zwar noch katalysiert, hinsichtlich der Weiterreaktion zu unerwünschtem Folgeprodukt jedoch inaktiver ist als der Zeolith-Katalysator in der ersten Reaktorzone.

Dabei ist es beispielsweise bevorzugt, in einem ersten Kompartiment des Reaktors einen Titanzeolithkatalysator vom Kristallstrukturtyp Ti-MWW mit einem hohen Titangehalt und demgemäß mit einer hohen Aktivität bezüglich der Umsetzung von Propen mit Hydroperoxid und in einem zweiten Kompartiment des Reaktors einen Titanzeolithkatalysator vom Kristallstrukturtyp Ti-MWW mit einem im Vergleich zum ersten Titanzeolithkatalysator niedrigeren Titangehalt einzusetzen. Gemäß einer ebenfalls bevorzugten Ausführungsform weisen die beiden Titanzeolithkatalysatoren unterschiedliche Kristallstruktur auf, wobei beispielsweise der Katalysator im ersten Kompartiment eine Kristallstruktur vom MFI-Typ und der Katalysator im zweiten Kompartiment eine Kristallstruktur vom MWW-Typ aufweist. Ebenso kann der Katalysator im ersten Kompartiment eine Kristallstruktur vom MWW-Typ und der Katalysator im zweiten Kompartiment eine Kristallstruktur vom MFI-Typ aufweisen. Im Rahmen dieser Ausführungsformen ist es besonders bevorzugt, dass die Reaktionsmischung beim Durchgang durch den Reaktor zunächst das erste Kompartiment und anschließend das zweite Kompartiment durchläuft.

Demgemäß beschreibt die vorliegende Erfindung auch den Reaktor zur Umsetzung von Propen mit einem Hydroperoxid, umfassend mindestens zwei räumlich voneinander getrennte, voneinander verschiedene Zeolith-Katalysatoren vom Kristallstrukturtyp MWW.

Die erfindungsgemäße Kompartimentierung kann im Rahmen der vorliegenden Erfindung beispielsweise auch durch den Einsatz von mindestens zwei seriell geschalteten Reaktoren realisiert werden, wobei in einem ersten Reaktor mindestens ein Zeolith-Katalysator zur Umsetzung verwendet wird und in mindestens einem weiteren Reaktor mindestens ein weiterer Zeolith-Katalysator zur Umsetzung zum Einsatz kommt, der sich von dem im ersten Reaktor eingesetzten Zeolith-Katalysator unterscheidet, wobei mindestens einer der Katalysatoren ein Zeolithkatalysator, bevorzugt ein Titanzeolithkatalysator vom Kristallstrukturtyp MWW ist.

Im Rahmen dieser Ausführungsform ist es beispielsweise möglich, in mindestens einem ersten Reaktor einen ersten Katalysator und in mindestens einem zweiten Reaktor einen zweiten, vom ersten Katalysator verschiedenen Katalysator einzusetzen, wobei der Katalysator im ersten Reaktor beispielsweise als zeolithisches Material oder als Formkörper oder als Mischung aus zeolithischem Material und Formkörper und der Katalysator im zweiten Reaktor als zeolithisches Material oder als Formkörper, oder als Mischung aus zeolithischem Material und Formkörper vorliegen können.

Ebenso kann der Katalysator beispielsweise als Netzkatalysator auf Basis von inerten Netzgeweben wie beispielsweise inerten Netzgeweben aus Metallen, Kunststoffen, Aluminiumoxiden, Glasfasern, Kohlefasern und/oder Graphit eingesetzt werden. Bezogen auf die Gewichtsmenge des Zeolithkatalysators weisen solche Netzkatalysatoren bevorzugt einen Alkalimetallgehalt von weniger als 500 ppm auf. Hergestellt werden diese Netzkatalysatoren bevorzugt gemäß einem Verfahren, bei dem mindestens ein Zeolith, bevorzugt mindestens ein Titanzeolith, auf inertes Netzgewebe aufkristallisiert wird. Netzkatalysatoren dieser Art und Wege zu ihrer Herstellung sind in der der EP 0 883 439 B1 beschrieben, deren diesbezüglicher Inhalt in den Kontext der vorliegenden Erfindung durch Bezugnahme vollumfänglich einbezogen wird.

Demgemäß beschreibt die vorliegende Erfindung auch einen seriell gekoppelten Reaktorverbund zur Umsetzung von Propen mit einem Hydroperoxid, umfassend mindestens zwei Reaktoren, der dadurch gekennzeichnet ist, dass mindestens zwei Reaktoren jeweils voneinander verschiedene Zeolith-Katalysatoren enthalten.

Als Zeolithkatalysatoren, die vom Zeolithkatalysator vom Kristallstrukturtyp MWW verschieden sind, sind im einzelnen Titan-, Germanium-, Tellur-, Vanadium-, Chrom-, Niob-, Zirkoniumhaltige Zeolithe mit Pentasil-Zeolith-Struktur, insbesondere die Typen mit röntgenografischer Zuordnung zur ABW-, ACO-, AEI-, AEL-, AEN-, AET-, AFG-, AFI-, AFN-, AFO-, AFR-, AFS-, AFT-, AFX-, AFY-, AHT-, ANA-, APC-, APD-, AST-, ATN-, ATO-, ATS-, ATT-, ATV-, AWO-, AWW-, BEA-, BIK-, BOG-, BPH-, BRE-, CAN-, CAS-, CFI-, CGF-, CGS-, CHA-, CHI-, CLO-, CON-, CZP-, DAC-, DDR-, DFO-, DFT-, DOH-, DON-, EAB-, EDI-, EMT-, EPI-, ERI-, ESV-, EUO-, FAU-, FER-, GIS-, GME-, GOO-, HEU-, IFR-, ISV-, ITE-, JBW-, KFI-, LAU-, LEV-, LIO-, LOS-, LOV-, LTA-, LTL-, LTN-, MAZ-, MEI-, MEL-, MEP-, MER-, MFI-, MFS-, MON-, MOR-, MSO-, MTF-, MTN-, MTT-, MTW-, NAT-, NES-, NON-, OFF-, OSI-, PAR-, PAU-, PHI-, RHO-, RON-, RSN-, RTE-, RTH-, RUT-, SAO-, SAT-, SBE-, SBS-, SBT-, SFF-, SGT-, SOD-, STF-, STI-, STT-, TER-, THO-, TON-, TSC-, VET-, VFI-, VNI-, VSV-, WIE-, WEN-, YUG-, ZON-Struktur sowie zu Mischstrukturen aus zwei oder mehr der vorgenannten Strukturen zu nennen. Denkbar sind für den Einsatz im erfindungsgemäßen Verfahren weiterhin titanhaltige Zeolithe mit der Struktur des ITQ-4, SSZ-24, TTM-1, UTD-1, CIT-1 oder CIT-5. Als weitere titanhaltige Zeolithe sind solche mit der Struktur des ZSM-48 oder ZSM-12 zu nennen.

Details hinsichtlich dieser Strukturtypen sind der oben genannten Literaturstelle W.M. Meier, D.H. Olson und Ch. Baerlocher "Atlas of Zeolite Structure Types", Elsevier, 5. Aufl., S. 202 und 203, Amsterdam 2001 zu entnehmen.

Titanzeolithe, insbesondere solche mit einer Kristallstruktur vom MFI-Typ, sowie Möglichkeiten zu ihrer Herstellung sind beispielsweise in der WO 98/55228, EP-A 0 311 983 oder der EP-A 0 405 978 beschrieben, deren diesbezüglicher Umfang vollumfänglich in den Kontext der vorliegenden Anmeldung einbezogen wird.

In diesem seriell gekoppelten Reaktorverbund kann beispielsweise in einem ersten Reaktor die Umsetzung in Anwesenheit eines ersten Katalysators vom TS-1-Typ und in einem zweiten Reaktor in Anwesenheit eines zweiten Katalysators vom MWW-Typ durchgeführt werden. Hierbei ist es beispielsweise bevorzugt, im ersten Reaktor als Lösungsmittel Methanol oder ein Methanol-Wasser-Gemisch und im zweiten Reaktor als Lösungsmittel mindestens ein Nitril, bevorzugt Acetonitril oder ein Nitril-, bevorzugt ein Acetonitril-Wassergemisch zu verwenden. Ebenso kann im ersten Reaktor der Katalysator vom MWW-Typ eingesetzt werden und im zweiten Reaktor der Katalysator vom TS-1-Typ.

Die folgenden Beispiele sollen die Erfindung näher erläutern.

## Beispiele

**Beispiel 1a** : Aktivitätstest mit TS-1 in Methanol als Lösungsmittel

In einem 21-Stahlautoklaven mit Rührer wurden 100 g calciniertes Titanzeolith-Sprühgranulat (Ti-Gehalt 1,5 Gew.-%), hergestellt gemäß dem Ausführungsbeispiel der DE-A 199 39 416, gemeinsam mit 108 g Ammoniaklösung (25 Gew.-%) und 972 g deionisiertem Wasser bei 300 U/min gerührt und für die Dauer von 24 h auf einer Temperatur von 125 °C gehalten.

Nach Beendigung der Reaktion wurde der Reaktorinhalt über eine Nutsche abgesaugt und dreimal mit insgesamt 1500 ml deionisiertem Wasser nachgewaschen.

Der Filterkuchen wurde 4 h bei 120 °C unter Luft getrocknet und abschließend 3 h bei 550 °C unter Luft calciniert.

Die Auswaage betrug 93 g, das Material zeigte einen Ti-Gehalt von 1,5 Gew.-%.

Im katalytischen Aktivitätstest wurden 0,5 g Titanzeolith TS-1, wie oben beschrieben hergestellt, mit 45 ml Methanol in einen druckfesten Glasreaktor gebracht, und bei 0 °C wurden 20 ml Propen zudosiert und anschließend über eine Pumpe 18 g Wasserstoffperoxid (Merck, 30 Gew.-% in Wasser) zugefahren. Nach 5 h Reaktionsdauer wurde das Gemisch entspannt und die flüssige Phase gaschromatographisch analysiert. Das Reaktionsgemisch enthielt 6,2 Gew.-% Propylenoxid.

**Beispiel 1b :** Aktivitätstest mit TS-1 in Acetonitril als Lösungsmittel

Beispiel 1a wurde wiederholt, wobei der katalytische Aktivitätstest mit Acetonitril statt Methanol als Lösungsmittel durchgeführt wurde.

Das Reaktionsgemisch enthielt 1,1 Gew.-% Propylenoxid.

**Beispiel 2a** : Aktivitätstest mit Ti-MWW in Acetonitril als Lösungsmittel

Die Herstellung wurde wie in Beispiel 1 der WO 02/28774 beschrieben durchgeführt. Dabei wurden 112 g Aerosil als Silica-Quelle eingesetzt. Die Auswaage an Ti-MWW betrug 70 g und das Material zeigte einen Ti-Gehalt von 4,6 Gew.-% sowie ein für Ti-MWW typisches Röngendiffraktogramm.

In einem katalytischen Aktivitätstest wurden 0,5 g Titanzeolith Ti-MWW mit 45 ml Acetonitril in einen druckfesten Glasreaktor gebracht, und bei 0 °C wurden 20 ml Propen zudosiert und anschließend über eine Pumpe 18 g Wasserstoffperoxid (Merck, 30 Gew.-% in Wasser) zugefahren. Nach 5 h Reaktionsdauer wurde das Gemisch entspannt und die flüssige Phase gaschromatographisch analysiert. Das Reaktionsgemisch enthielt 5,3 Gew.-% Propylenoxid.

**Beispiel 2b :** Aktivitätstest mit Ti-MWW in Methanol als Lösungsmittel

Beispiel 2a wurde wiederholt, wobei der katalytische Aktivitätstest mit Methanol statt Acetonitril als Lösungsmittel durchgeführt wurde.

Das Reaktionsgemisch enthielt 1,9 Gew.-% Propylenoxid.

**Beispiel 3 :**

35 g des gemäß Beispiel 2a hergestellten Ti-MWW-Pulvers wurden mit 45 g Kieselsol (Ludox® AS 40, 40 Gew.-% Siliciumdioxid) und insgesamt 20 g Polystyroldispersion (33,5 Gew.-% in Wasser) sowie 1,5 g Methylcellulose (Walocel®) und 45 g Wasser in einem Kneter gut vermischt.
Dabei wurden während des Knetens kontinuierlich innerhalb 1 min die Polystyroldispersion und nach 3 min langsam 37,5 g Ludox® zugegeben. Nach weiteren 2 min Kneten wurden 10 g Wasser zugegeben und nach weiteren 10 min nochmals langsam 15 g Wasser. Nach weiteren 10 min wurde das restliche Kieselsol und anschließend innerhalb 10 min portionsweise das restliche Wasser zugegeben. Methylcellulose wurde zusammen mit dem trockenen Pulver im Kneter zuerst gemischt.
Die entstandene Paste wurde nach insgesamt 70 min Knetzeit bei einem Pressdruck von 60 bar über eine Strangpresse mit einer Matrize von 1,5 mm-Löchern zu Strängen ausgeformt.
Das so erhaltene Produkt wurde 4 h bei 120 °C unter Luft und bei einer Aufheizrate von 2 °C/min getrocknet. Anschließend wurde bei 490 °C für 5 h und einer Aufheizrate von 1 °C/min unter Luft calciniert. Die Ausbeute betrug 41 g.
Der Titangehalt des so hergestellten Katalysators lag bei 2,8 Gew.-% und das mittels Quecksilberporosimetrie nach DIN 66133 ermittelte Porenvolumen betrug 1,2 ml/g.

**Beispiel 4 :**

Von dem gemäß Beispiel 3 erhaltenen Katalysator wurden 15,1 g in einem Rohrreaktor von 1,3 m Länge eingebaut und bei 20 bar Druck Acetonitril mit einem Feed von 66 g/h, Wasserstoffperoxid (40 Gew.-%) mit einem Feed von 8,9 g/h und Propen (96 Vol.-% Propen) mit einem Feed von 7 g/h bei Temperaturen im Bereich von 30 bis 60 °C beaufschlagt.
Die Analyse des aus dem Reaktor austretenden Produktgemisches ergab bei einer kontinuierlichen Laufzeit von 400 Stunden eine mittlere Selektivität zu Propylenoxid, bezogen auf Wasserstoffperoxid, von 81,5 % und eine mittlere Selektivität zu Epoxidfolgeprodukten, bezogen auf Wasserstoffperoxid, von 4,7 %. Die mittlere Selektivität zu Glykol als Epoxidfolgeprodukte lag bei 1,4 %. Die Selektivität hinsichtlich Sauerstoff betrug im Mittel 13,8 %.

**Beispiel 5:**

9 g des Katalysators aus Beispiel 3 wurden mit 100 g deionisiertem Wasser in einem Autoklaven unter Rühren auf 140 °C aufgeheizt und für die Dauer von 36 Stunden gerührt.
Anschließend wurde der Ansatz abgekühlt, der Feststoff abfiltriert, bei 120 °C für 4 Stunden getrocknet und bei 450 °C unter Luft calciniert. Die Auswaage betrug 8,8 g.

**Beispiel 6:**

Von dem in Beispiel 5 erhaltenen Katalysator wurden 8,8 g in einen Rohrreaktor (1,3 m Länge) eingebaut und bei 20 bar mit einem Feed aus 42 g/h Acetonitril, 5,5 g/h Wasserstoffperoxid (40 Gew.-%) und 4,5 g/h Propen (96 Vol.-% Propen) bei Temperaturen zwischen 30 und 65°C beaufschlagt.
Die Analyse des aus dem Reaktor austretenden Produktgemisches ergab bei einer kontinuierlichen Laufzeit von 325 Stunden eine mittlere Selektivität zu Propylenoxid, bezogen auf Wasserstoffperoxid, von 87,6 % und eine mittlere Selektivität zu Epoxidfolgeprodukten, bezogen auf Wasserstoffperoxid, von 6,0 %. Die mittlere Selektivität zu Glykol als Epoxidfolgeprodukte lag bei 1,6 %. Die Selektivität hinsichtlich Sauerstoff betrug im Mittel 6,5 %.

**Patentansprüche**

1. Verfahren zur Herstellung von Propylenoxid, mindestens umfassend die Schritte (i) und (ii):

(i) Bereitstellen eines Katalysators, enthaltend mindestens ein poröses oxidisches Material;
(ii) Umsetzung von Propen mit einem Hydroperoxid in mindestens einem Nitril als Lösungsmittel oder in einem mindestens ein Nitril enthaltenden Lösungsmittelgemisch in Anwesenheit des Katalysators gemäß (i),

**dadurch gekennzeichnet, dass** das mindestens ein poröse oxidische Material ein Zeolith mit röntgenographischer Zuordnung zum MWW-Typ ist.

**2.** Verfahren nach Anspruch 1, **dadurch gekennzeichnet, dass** die Selektivität hinsichtlich der Epoxidfolgeprodukte, bezogen auf Hydroperoxid, höchstens 10 % beträgt.

**3.** Verfahren nach Anspruch 1 oder 2, **dadurch gekennzeichnet, dass** der Zeolith vom MWW-Typ ein Titanzeolith ist.

**4.** Verfahren nach einem der Ansprüche 1 bis 3, **dadurch gekennzeichnet, dass** die Bereitstellung des Katalysators gemäß (i) mindestens die Schritte (a) und (b) umfasst:

(a) Herstellung des mindestens einen porösen oxidischen Materials;
(b) Herstellung eines Formkörpers unter Verwendung des gemäß (a) erhaltenen porösen oxidischen Materials.

**5.** Verfahren nach Anspruch 4, **dadurch gekennzeichnet, dass** die Herstellung des Formkörpers gemäß (b) mindestens die Schritte (aa) bis (dd) umfasst:

(aa) Kneten des gemäß (a) erhaltenen porösen oxidischen Materials unter Zugabe mindestens eines Bindemittels oder mindestens eines Verstrangungshilfsmittels oder mindestens eines Porenbildners oder einer Wasser enthaltenden Zusammensetzung oder eines Gemischs aus zwei oder mehr davon;
(bb) Verformen der gemäß (aa) erhaltenen gekneteten Mischung unter Erhalt mindestens eines Formkörpers;
(cc) Trocknen des gemäß (bb) erhaltenen Formkörpers;
(dd) Calcinieren des gemäß (cc) erhaltenen getrockneten Formkörpers.

**6.** Verfahren nach einem der Ansprüche 1 bis 5, **dadurch gekennzeichnet, dass** das Hydroperoxid Wasserstoffperoxid und das mindestens eine Nitril Acetonitril ist.

**7.** Verfahren nach einem der Ansprüche 1 bis 6, **dadurch gekennzeichnet, dass** die Umsetzung gemäß (ii) in mindestens einem kontinuierlich betriebenen Reaktor bei Temperaturen im Bereich von 20 bis 80 °C und bei Drücken im Bereich von 15 bis 45 bar durchgeführt wird.

**8.** Katalysatorformkörper, als katalytisch aktives Material mindestens enthaltend ein poröses oxidisches Material, **dadurch gekennzeichnet, dass** das poröse oxidische Material ein Zeolith mit röntgenographischer Zuordnung zum MWW-Typ ist.

**9.** Katalysatorformkörper nach Anspruch 8, **dadurch gekennzeichnet, dass** der Durchmesser von 80 % der bimodal verteilten Poren des Formkörpers im Bereich von 4 nm bis 1 $\mu$m liegt.

**10.** Verwendung eines Titanzeolithen mit röntgenographischer Zuordnung zum MWW-Typ zur Epoxidierung von Propen mit einem Hydroperoxid.

Nummer der Anmeldung

EP 10 17 3947

Europäisches
Patentamt

European
Patent Office

Office européen
des brevets

**EUROPÄISCHER RECHERCHENBERICHT**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 02/28774 A (OGUCHI WATARU ; WU PENG (JP); SHOWA DENKO KK (JP); TATSUMI TAKASHI (JP) 11. April 2002 (2002-04-11) | 8,9 | INV.<br>C07D301/12<br>C01B37/00 |
| Y | * Seite 25, Zeilen 23-34 *<br>* Seite 1, Zeilen 22-34; Anspruch 20; Beispiel 6 *<br>* Seite 26, Zeilen 2-13 *<br>----- | 1-7,10 | |
| Y | WU P ET AL: "A Novel Titanosilicate with MWW Structure: II. Catalytic Properties in the Selective Oxidation of Alkenes"<br>JOURNAL OF CATALYSIS, ACADEMIC PRESS, DULUTH, MN, US,<br>Bd. 202, Nr. 2,<br>10. September 2001 (2001-09-10), Seiten 245-255, XP004432448<br>ISSN: 0021-9517<br>* Seiten 245-248, *<br>* Seite 255 *<br>----- | 1-3,6,10 | |
| Y | WU P ET AL: "Extremely high trans selectivity of Ti-MWW in epoxidation of alkenes with hydrogen peroxide"<br>CHEMICAL COMMUNICATIONS - CHEMCOM, ROYAL SOCIETY OF CHEMISTRY, GB, Nr. 10, 2001, Seiten 897-898, XP002962167<br>ISSN: 1359-7345<br>* das ganze Dokument *<br>----- | 1-3,6,10 | **RECHERCHIERTE SACHGEBIETE (IPC)**<br><br>C07D<br>C01B |
| Y | US 3 393 156 A (HANSFORD ROWLAND C) 16. Juli 1968 (1968-07-16)<br>* Spalte 1, Zeilen 12-50 *<br>----- | 4,5 | |

-/--

Der vorliegende Recherchenbericht wurde für alle Patentansprüche erstellt

| Recherchenort | Abschlußdatum der Recherche | Prüfer |
|---|---|---|
| München | 13. Oktober 2010 | Rudolf, Manfred |

KATEGORIE DER GENANNTEN DOKUMENTE

X : von besonderer Bedeutung allein betrachtet
Y : von besonderer Bedeutung in Verbindung mit einer
      anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
      nach dem Anmeldedatum veröffentlicht worden ist
D : in der Anmeldung angeführtes Dokument
L : aus anderen Gründen angeführtes Dokument
...................................................................................................
& : Mitglied der gleichen Patentfamilie, übereinstimmendes
      Dokument

EPO FORM 1503 03.82 (P04C03)

**Europäisches Patentamt**
**European Patent Office**
**Office européen des brevets**

# EUROPÄISCHER RECHERCHENBERICHT

**Nummer der Anmeldung**

EP 10 17 3947

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 6 491 861 B1 (GROSCH GEORG HEINRICH ET AL) 10. Dezember 2002 (2002-12-10) <br> * Spalte 1, Zeilen 16-33 * <br> * Spalte 2, Zeile 40 - Spalte 3, Zeile 21 * <br> * Spalte 6, Zeilen 44-51 * <br> ----- | 4,5 | |
| Y | US 6 066 750 A (CHANG TE) 23. Mai 2000 (2000-05-23) <br> * Spalte 1, Zeilen 8-18 * <br> * Spalte 2, Zeilen 39-65 * <br> * Spalte 4, Zeilen 1-51,66 * <br> * Spalte 5, Zeilen 22-55; Beispiele 1,2 * <br> ----- | 1-3,6,7, 10 | |
| Y | US 6 008 389 A (GROSCH GEORG HEINRICH ET AL) 28. Dezember 1999 (1999-12-28) <br> * Spalte 1, Zeilen 20-27 * <br> * Spalte 2, Zeile 7 - Spalte 3, Zeile 3 * <br> * Spalte 4, Zeilen 21,22 * <br> ----- | 1-7,10 | |

**RECHERCHIERTE SACHGEBIETE (IPC)**

Der vorliegende Recherchenbericht wurde für alle Patentansprüche erstellt

| Recherchenort | Abschlußdatum der Recherche | Prüfer |
|---|---|---|
| München | 13. Oktober 2010 | Rudolf, Manfred |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument

........................................................................

& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 10 17 3947

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

13-10-2010

| Im Recherchenbericht angeführtes Patentdokument | | Datum der Veröffentlichung | Mitglied(er) der Patentfamilie | | Datum der Veröffentlichung |
|---|---|---|---|---|---|
| WO 0228774 | A | 11-04-2002 | AU | 9029501 A | 15-04-2002 |
| | | | CA | 2423666 A1 | 11-04-2002 |
| | | | CN | 1466545 A | 07-01-2004 |
| US 3393156 | A | 16-07-1968 | DE | 1542536 A1 | 16-04-1970 |
| | | | GB | 1102518 A | 07-02-1968 |
| US 6491861 | B1 | 10-12-2002 | AT | 213664 T | 15-03-2002 |
| | | | AU | 8336298 A | 21-12-1998 |
| | | | CA | 2293617 A1 | 10-12-1998 |
| | | | CN | 1266382 A | 13-09-2000 |
| | | | DE | 19723751 A1 | 10-12-1998 |
| | | | DE | 59803203 D1 | 04-04-2002 |
| | | | WO | 9855229 A1 | 10-12-1998 |
| | | | EP | 0991469 A1 | 12-04-2000 |
| | | | ES | 2173599 T3 | 16-10-2002 |
| | | | ID | 23432 A | 20-04-2000 |
| | | | JP | 2002504013 T | 05-02-2002 |
| | | | KR | 20010013467 A | 26-02-2001 |
| | | | MX | 215974 B | 21-08-2003 |
| US 6066750 | A | 23-05-2000 | AU | 8730598 A | 25-01-1999 |
| | | | BR | 9810377 A | 05-09-2000 |
| | | | CA | 2293637 A1 | 14-01-1999 |
| | | | CN | 1261359 A | 26-07-2000 |
| | | | DE | 69808091 D1 | 24-10-2002 |
| | | | DE | 69808091 T2 | 30-04-2003 |
| | | | WO | 9901445 A1 | 14-01-1999 |
| | | | EP | 0993453 A1 | 19-04-2000 |
| | | | ES | 2330831 T3 | 16-12-2009 |
| | | | ES | 2184312 T3 | 01-04-2003 |
| | | | JP | 4301580 B2 | 22-07-2009 |
| | | | JP | 2002508779 T | 19-03-2002 |
| | | | US | 5912367 A | 15-06-1999 |
| US 6008389 | A | 28-12-1999 | AU | 3030997 A | 07-01-1998 |
| | | | BR | 9709702 A | 10-08-1999 |
| | | | CA | 2256383 A1 | 18-12-1997 |
| | | | CN | 1222096 A | 07-07-1999 |
| | | | DE | 19623609 A1 | 18-12-1997 |
| | | | WO | 9747386 A1 | 18-12-1997 |
| | | | EP | 0904151 A1 | 31-03-1999 |
| | | | ES | 2162300 T3 | 16-12-2001 |
| | | | JP | 2000511818 T | 12-09-2000 |
| | | | KR | 20000016574 A | 25-03-2000 |

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0228774 A2
- DE 10232406
- WO 9855229 A
- EP 0389041 A
- EP 0200260 A
- WO 9519222 A
- DE 19835907 A
- EP 0883439 B1
- WO 9855228 A
- EP 0311983 A
- EP 0405978 A
- DE 19939416 A
- WO 0228774 A

**In der Beschreibung aufgeführte Nicht-Patentliteratur**

- **Wu et al.** Hydrothermal Synthesis of a novel Titanosilicate with MWW Topology. *Chemistry Letters,* 2000, 774-775
- **W.M. Meier ; D.H. Olson ; Ch. Baerlocher.** Atlas of Zeolite Structure Types. Elsevier, 2001
- **W.M. Meier ; D.H. Olson ; Ch. Baerlocher.** Atlas of Zeolite Structure Types. Elsevier, 2001, 202, 203